## Europäisches Patentamt

## European Patent Office

## Office européen des brevets

(11) Veröffentlichungsnummer: **0 212 518 A2**

(12) **EUROPÄISCHE PATENTANMELDUNG**

(21) Anmeldenummer: 86111037.7

(22) Anmeldetag: 09.08.86

(51) Int. Cl.⁴: **C 12 P 7/06**, C 13 K 3/00
// (C12P7/06, C12R1:01)

(30) Priorität: 13.08.85 DE 3528933

(43) Veröffentlichungstag der Anmeldung: 04.03.87
Patentblatt 87/10

(84) Benannte Vertragsstaaten: AT BE DE FR GB IT NL SE

(71) Anmelder: Kernforschungsanlage Jülich Gesellschaft mit beschränkter Haftung, Postfach 1913, D-5170 Jülich 1 (DE)

(72) Erfinder: Bringer-Meyer, Stefanie, Dr., Buschhelmer Strasse 23, D-4150 Krefeld-Bockum (DE)
Erfinder: Sahm, Hermann, Prof., Wendelinusstrasse 71, D-5170 Jülich (DE)

(54) Fermentationsverfahren zur Fructosegewinnung oder -anreicherung gegenüber Glucose und dafür brauchbare Zymomonas mobilis Mutanten.

(57) Die Gewinnung von Fructose bzw. deren Anreicherung gegenüber Glucose ausgehend von einer Fructose und Glucose enthaltenden wäßrigen Mischung durch fermentativen Glucoseabbau zu Ethanol und Kohlendioxid erfolgt mittels einer durch kontinuierliche Kultur über zumindest 10 Tage, insbesondere 100 Tage erhaltenen stabilen fructose-negativen Mutante von Zymomonas mobilis. Insbesondere wird die bei der Deutschen Sammlung von Mikroorganismen in Göttingen hinterlegte Mutante mit der Hinterlegungsnummer DSM 3126 verwendet. Zuckerkonzentrationen der wäßrigen Ausgangsmischungen mit je $\geq$ 1% bzw. je 4 bis 13% und insbesondere je 7,5 bis 10% Glucose und Fructose sind geeignet. Die Fermentation erfolgt insbesondere kontinuierlich.

Kernforschungsanlage Jülich
Gesellschaft mit beschränkter Haftung

Fermentationsverfahren zur Fructosegewinnung oder -anreicherung gegenüber
Glucose und dafür brauchbare Zymomonas
mobilis Mutanten

Die Erfindung bezieht sich auf ein Verfahren zur Fructosegewinnung oder -anreicherung gegenüber Glucose ausgehend von einer Fructose und Glucose enthaltenden wässrigen Mischung durch Fermentation mittels einer Fructose nicht abbauenden Mutante von Zymomonas mobilis zur Umwandlung von Glucose in Ethanol und Kohlendioxid, sowie auf dafür brauchbare Zymomonas mobilis Mutanten.

Fructose ist wegen ihrer um das Anderthalbfache höheren Süßkraft im Vergleich zu Saccharose ein wertvolles Süßungsmittel, das hauptsächlich für pharmazeutische Zwecke und für kalorienarme Nahrungsmittel verwendet wird.
Gebräuchliche Verfahren zur Gewinnung der Fructose gehen von Invertzucker oder HFCS[*] aus, d.h. Glucose/Fructose-Mischungen, aus denen die Fructose durch Trennverfahren wie Kristallisation, Fällung oder Chromatographie ggf. unter Komplexierung z.B. mit Borsäure oder Umwandlung der Glucose in Gluconsäure gewonnen wird. Diese Trennver-

[*] High Fructose Corn Syrup

- 2 -

fahren sind wegen der Ähnlichkeit beider Zucker relativ aufwendig, und es wurden daher auch bereits Versuche unternommen, enzymatische bzw. mikrobielle Verfahren zu entwickeln.

So wird in der US-PS 4 356 262 ein Verfahren zur Gewinnung von Fructosesirup und Ethanol aus Saccharoselösung beschrieben, bei dem zunächst mittels Fructosyltransferase von Pullularia pullulans und Hefen wie Saccharomyces bailii oder cerevisiae, Fructosepolymere und Ethanol gebildet werden, die dann nachträglich zu Fructose hydrolysiert werden können. Dieses Verfahren umfaßt jedoch, ausgehend von Saccharose, mehrere komplizierte Vorbereitungs- und Reaktionsschritte. Zur Bereitstellung des für die Transfructosylierung notwendigen Enzyms muß ein spezieller Mikroorganismus vorkultiviert und präpariert werden. Die gleichzeitig mit dem Enzym oder in einem gesonderten Schritt zugesetzte Gärhefe wandelt dann die direkt anfallende Glucose zu Ethanol um. Die technisch besonders interessante Fructose fällt verfahrensgemäß nur als Polymeres an und muß unter diffizilen Bedingungen und nachträglich in einem weiteren Schritt hydrolytisch gespalten werden. Technisch erscheint ein solch kompliziertes Verfahren zur Gewinnung von Fructose als viel zu kostspielig.

In der AU-A 29 530/84 wird ein Verfahren zur Herstellung von Fructose und Ethanol aus Saccharose beschrieben, wobei eine Saccharosekonzentration $\geq$ 30 gew. % vorliegen soll. Mit Hilfe des Bakteriums Zymomonas mobilis, vorzugsweise nach Vorbehandlung der Saccharoselösung mit einer immobilisierten Levansucrase-Enzympräparation (EC 2.4.1.10), soll eine selektive Umsetzung der Glucose zu Ethanol innerhalb von 1-2 Tagen erfolgen. Dieses Verfahren weist jedoch ebenfalls erhebliche Nachteile auf: das Enzym Levansucrase ist gemäß EC 2.4.1.10 als Fructosyltransferase wirksam (T.E. Barman

— 3 —

(1969) Enzyme Handbook I, S. 310), d.h. die Reaktionsprodukte von Saccharose sind Polyfructose (Levan) und Glucose. Außerdem ist bekannt, daß auch Zymomonas aus Saccharose Levan produziert (D.W. Ribbons (1962) Biochem. J. $\underline{82}$, 45 P; E.A. Dawes et al. (1966) Biochem. J. $\underline{98}$, 804; K.J. Lee (1981) Biotechnol. Lett. $\underline{3}$, 207). Die verfahrensgemäßen Maßnahmen nach der AU-A führen daher auch zu Levan- und damit zu erheblicher Schleimbildung. Die Bildung von Levan hat natürlich auch Verluste an monomerer Fructose zur Folge. Weiterhin setzen bekannte Stämme von Zymomonas mobilis Fructose auch zu Ethanol um (Swings und DeLey (1977) Bacteriol. Rev. $\underline{41}$, 1), was die Fructoseausbeute in obigem Verfahren zusätzlich vermindern muß.

Über Versuche zur Gewinnung Fructose-negativer Mutanten von Zymomonas mobilis wurde bereits auf dem 11. Int. Carbohydrate Symposium in Vancouver (S. Bringer, M. Scollar, H. Sahm (1982) Referat 39 der Abstracts) vorgetragen. Die dafür angegebenen Mutanten erwiesen sich jedoch für eine Praxisanwendung zur Anreicherung von Fructose aus Fructose/Glucose-Mischungen als ungeeignet, da die Mutanten zu instabil waren und schon nach kurzer Zeit unvorhersehbar ihre Selektivität wieder verloren.

Nach den bekannten, teilweise sehr aufwendigen Verfahren werden somit große Anteile von wenig erwünschten Nebenprodukten produziert oder die anfänglich geeigneten Mikroorganismen sind nur zu kurzzeitig stabil.

Ziel der Erfindung ist daher ein Fructosegewinnungsverfahren, das auch über lange Zeiten hinweg verläßlich zu hohen Fructoseausbeuten führt.

Das zu diesem Zweck entwickelte erfindungsgemäße Verfahren der eingangs genannten Art ist dadurch gekennzeichnet, daß man die Fermentation mittels einer durch durch kontinuierliche Kultur über zumindest 10 Tage, insbesondere 100 Tage erhaltenen stabilen Mutante von Zymomonas mobilis durchführt.

Überraschend wurde nämlich gefunden, daß aus Glucose und Fructose enthaltenden wäßrigen Mischungen Fructose in hohen Ausbeuten erhalten werden kann, wenn man die Vergärung von Glucose zu Ethanol mit stabilen, Fructose-negativen Mutanten von Zymomonas mobilis, die mindestens 10 Tage, vorzugsweise mindestens 100 Tage die Fructose-Negativität unverändert beibehalten, durchführt. Neben der einfachen Durchführbarkeit dieses einstufigen Verfahrens ist es besonders vorteilhaft, daß keine Fructosepolymere anfallen, die durch Schleimbildung die Aufarbeitung erschweren.

Vorzugsweise erfolgt diese Fructosegewinnung oder -anreicherung aus wäßrigen Mischungen, die mindestens je 1 %, vorzugsweise je 4 bis 13 % und insbesondere je 7,5 bis 10 % Glucose und Fructose enthalten, wobei das Verfahren sowohl batch- und fed batch-weise als auch kontinuierlich durchgeführt werden kann.

Die Isolierung der Fructose aus der Reaktionsmischung kann nach bekannten Verfahren, z.B. durch Chromatographie, oder fraktionierte Fällung vorgenommen werden.

Die Erfindung umfaßt auch spezielle neue stabile Mutanten von Zymomonas mobilis, die in Glucose/Fructose-Mischungen nur aus Glucose, nicht aber aus Fructose Ethanol und $CO_2$ produzieren und diese Eigenschaft über mindestens 10 Tage behalten.

Sie umfaßt insbesondere die bei der Deutschen Sammlung von Mikroorganismen in Göttingen hinterlegte Mutante mit der Hinterlegungsnummer DSM 3126.

Die %-Angaben sind, soweit nicht anders benannt, immer Gewichts-%.

Die Verwendung niedriger Zuckerkonzentrationen ( je 1-4 % Fructose und Glucose) hat den Vorteil, daß die Glucosevergärung schnell abläuft und die Fructose nahezu quantitativ zurückbleibt. Verwendet man hingegen hochkonzentrierte Zuckerlösungen (z.B. je $\geq$ 20 % Fructose und Glucose) sind die Reaktionszeiten um ein Vielfaches länger und die Fructoseausbeute sinkt. Nach dem erfindungsgemäßen Verfahren fällt, insbesondere bei höheren Konzentrationen der Ausgangslösung, zunehmend als einziges aus der Fructose stammendes Nebenprodukt Sorbit an, ein Reduktionsprodukt von Fructose, welches gleichfalls in der Lebensmittelindustrie als Zuckeraustauschstoff, vorzugsweise für diätische Produkte, von großem Interesse ist. Für die Gewinnung von überwiegend Fructose- und Ethanol-haltigen Produktlösungen mit Hilfe der erfindungsgemäßen Mutanten sind also Substratkonzentrationen von je 7,5-10 % Glucose und Fructose besonders zu bevorzugen.

Um die Reduktion von Fructose zu Sorbit durch Zymomonas mobilis, die in nennenswertem Ausmaß nur in Gegenwart hoher Glucosekonzentrationen erfolgt zu reduzieren, kann die Fructoseausbeute erfindungsgemäß noch durch die folgenden Fermentationsmethoden, bei denen die Glucosekonzentration niedrig gehalten wird, erhöht werden. Besonders geeignet sind hierfür fed-batch und kontinuierliche Systeme. So werden in fed-batch Fermentationen, bei denen sukzessive, jeweils nach Vergärung der anfänglich eingesetzten Substratlösung diese Anfangskonzentration durch Zugabe von konzentrierter Fructose/Glucose Lösung (z.B. je 40 %-ig) wieder eingestellt wird, hohe Glucosekonzentrationen vermieden. Hierbei können mit Hilfe der erfindungsgemäßen Mutanten bei z.B. 10-maliger Nachdosierung von Substratlösung zu einer Kultur mit einer Anfangskonzentration von je 2 % Glucose und Fructose Produktlösungen erhalten werden, die 12-15 % Fructose und 7,5-10 vol.-% Ethanol enthalten. Auch kontinuierliche Fermentationen, die unter Glucoselimitierung und mit Substratkonzentrationen von je 5-13 %, bevorzugt je 10-12 % Glucose und Fructose durchgeführt werden, führen zu ähnlich hohen Fructoseausbeuten. Diese beiden Methoden, fed-batch und kontinuierliche Fermentation, sind nur mit den erfindungsgemäßen Mutanten für die Fructosegegwinnung aus Glucose/Fructose-Mischungen erfolgreich anwendbar, da die Wildstämme von Zymomonas mobilis Fructose nahezu quantitativ zu Ethanol vergären, wenn die gleichzeitig vorliegenden Glucosekonzentrationen niedrig sind.

Die Induktion der erfindungsgemäßen Mutanten in Wildstämmen von Zymomonas mobilis kann durch Bestrahlung der Zellen mit ultraviolettem Licht, vorzugsweise durch chemische Mutagenese mit nicht-alkylierenden Agentien wie Nitrit, Bisulfit, Hydroxylamin, oder mit alkylierenden Agentien wie Alkylalkansulfonate (z.B. Ethylmethansulfonat, Ethylethansulfonat, Diethylsulfat) und N-Nitroso-Verbindungen (z.B. Dialkyl-Nitrosamine, N-Nitrosoharnstoffe, N-Alkyl-N'-nitro-N-nitrosoguanidine), besonders bevorzugt durch das alkylierende Agenz N-Methyl-N'-nitro-N-nitrosoguanidin erfolgen.

Für die Kultivierung der erfindungsgemäßen Mutanten eignen sich Medien, die die folgenden Bestandteile enthalten (Mengen in g/l $H_2O$): $KH_2PO_4$: 0,5-1,0; $(NH_4)_2SO_4$: 0,5-1,0; $MgSO_4 \times 7H_2O$: 0,5-0,75; Hefeextrakt: 5-10; vergärbaren Zucker (Glucose); 10-130; der pH-Wert des Mediums sollte zu Beginn und während der Fermentation zwischen 4 und 7 liegen, die Temperatur zwischen 25° und 37°C, vorzugsweise von 28-32 °C.

Weitere Besonderheiten gehen aus der nachfolgenden Beschreibung der Erfindung anhand von Beispielen hervor.

1) Induktion und Selektion Fructose-negativer stabiler Mutanten

Eine Zellsuspension des Wildstammes Zymomonas mobilis ATCC 29191 wurde mit einer Zelldichte von $2,8 \times 10^{8}$ Lebendzellen/ml, entsprechend einer optischen Dichte ($OD_{550\ nm}$) von 7,0, in 0,1 M Kaliumphosphatpuffer, pH 6,0, mit $1,9 \times 10^{-4}$ M N-Methyl-N'-nitro-N-nitrosoguanidin (NTG) suspendiert. Nach 15-minütiger Inkubation bei Raumtemperatur wurden die Zellen zweimal mit 0,9 % NaCl-Lösung gewaschen. Zur Mutations-Ausprägung folgte eine 20-stündige Inkubation in Flüssigmedium (g/l $H_2O$: $KH_2PO_4$: 1,0; $(NH_4)_2SO_4$: 1,0; $MgSO_4 \times 7H_2O$: 0,5; Hefeextrakt: 5; pH = 5,0) mit 2 % Glucose bei 30°C. Eine anschließende Inkubation in Flüssigmedium mit 2 % Fructose + 0,3 mg/ml Ampicillin diente der Mutantenanreicherung. Diese Ausprägungs- und Anreicherungsphasen wurden dreimal wiederholt. Fructose-negative Mutanten wurden durch Replikaplattierungen auf Glucose- bzw. Fructose-enthaltenden Agarplatten (Flüssigmedium + 2 % Agar) identifiziert und isoliert.

Die Stabilität der Fructose-negativen Mutanten wurde durch Ausplattierung unverdünnter Kulturproben auf Fructose-enthaltenden Agarplatten (Flüssigmedium + 2 % Agar) überprüft. Kulturen der Mutante DSM 3126 blieben während und nach einem Zeitraum von 6 Monaten, bei 2-7-tägigen Überimpfungszeiträumen, in Flüssigkultur stabil. Ebenso blieb die Fructose-Negativität dieser Mutante während des Wachstums in kontinuierlicher Kultur über 5 Tage unverändert erhalten.

2) <u>Fructosegewinnung</u>

<u>Beispiel 1</u>

Ein Medium bestehend aus 18,2 g/l Fructose und 20,6 g/l Glucose, gelöst in dem unter 1) beschriebenen Flüssigmedium wurde in einem pH- und Temperatur-regulierten Fermenter mit 10 % (v/v) einer auf dem gleichen Medium gewachsenen Vorkultur der Mutante Zymomonas mobilis DSM 3126 beimpft. Vom Zeitpunkt des Animpfens bis zum Ende des Fermenterlaufs wurde der pH-Wert auf 5,0 und die Temperatur bei 30°C gehalten. Die Ergebnisse sind in Tabelle 1 angegeben.

<u>Beispiel 2-3</u>

Die Fermentationen wurden wie Beispiel 1 durchgeführt, jedoch unter Variation der Anfangs-Zuckerkonzentrationen. Die Ergebnisse sind in Tabelle 1 zusammengestellt.

Wie man sieht, wird in relativ verdünnter Zuckerlösung Glucose relativ rasch praktisch vollständig abgebaut, während eine erhebliche Umsetzung der Fructose zu Sorbit erst bei höheren Fructosekonzentrationen und längeren Gärzeiten auftritt.

## Tabelle 1

|  | Beispiel 1 | Beispiel 2 | Beispiel 3 |
|---|---|---|---|
| __Anfangskonzentrationen:__* |  |  |  |
| Fructose (g/l) | 18,2 | 48,3 | 98,5 |
| Glucose (g/l) | 20,6 | 49,9 | 100,2 |
| __Endkonzentrationen:__* |  |  |  |
| Fructose (g/l) | 15,9 | 33,9 | 72,7 |
| Glucose (g/l) | 0,0 | 0,0 | 0,6 |
| Ethanol (g/l) | 8,2 | 22,0 | 42,5 |
| Sorbit (g/l) | 2,1 | 10,6 | 24,5 |
| __Fermentationszeit (h)__ | 8 | 11 | 30 |
| __Fructoseanreicherung:__ |  |  |  |
| [Fructose]:[Gesamtzucker] (%) | 100 | 100 | 99 |

*10 g/l $\triangleq$ 1 Gew.-%

Kernforschungsanlage Jülich
Gesellschaft mit beschränkter Haftung

P a t e n t a n s p r ü c h e

1. Verfahren zur Fructosegewinnung oder -anreicherung gegenüber Glucose ausgehend von einer Fructose und Glucose enthaltenden wässrigen Mischung durch Fermentation mittels einer Fructose nicht abbauenden Mutante von Zymomonas mobilis zur Umwandlung von Glucose in Ethanol und Kohlendioxid, d a d u r c h   g e k e n n z e i c h - n e t,  daß man die Fermentation mittels einer durch kontinuierliche Kultur über zumindest 10 Tage, insbesondere 100 Tage erhaltenen stabilen Mutante von Zymomonas mobilis durchführt.

2. Verfahren nach Anspruch 1, d a d u r c h   g e k e n n z e i c h n e t,  daß man die bei der Deutschen Sammlung von Mikroorganismen in Göttingen hinterlegte Mutante mit der Hinterlegungsnummer DSM 3126 verwendet.

3. Verfahren nach Anspruch 1 oder 2, d a d u r c h   g e k e n n z e i c h n e t,  daß die Zuckerkonzentration der wäßrigen Mischungen mindestens je 1 %, vorzugsweise je 4 bis 13 % und insbesondere je 7,5 bis 10 % Glucose und Fructose betragen.

4. Verfahren nach einem der vorangehenden Ansprüche, d a d u r c h   g e k e n n z e i c h n e t,  daß die Fermentation kontinuierlich durchgeführt wird.

PT 1.784
nö/Fr

0212518

5. Zum selektiven Abbau von Glucose aus Glucose/ Fructose-Mischungen befähigte stabile Mutanten von Zymomonas mobilis erhältlich durch Mutation eines Wildstammes von Zymomonas mobilis durch physikalische und/oder chemische mutagene Mittel, Isolierung der fructosenegativen Mutanten und Selektion der stabilen Mutanten mittels kontinuierlicher Kultur über zumindest 10 Tage.

6. Zymomonas Mutante nach Anspruch 5, d a d u r c h g e k e n n z e i c h n e t, daß die Mutation durch Behandlung mit N-Methyl-N'-nitro-N-nitrosoguanidin herbeigeführt worden ist.

7. Zymomonas Mutante nach Anspruch 5, g e k e n n - z e i c h n e t d u r c h die Hinterlegungsnummer DSM 3126.